# EUROPEAN PATENT APPLICATION

(11) **EP 0 984 009 A1**
(43) Date of publication of application: **08.03.2000**
(21) Application number: 99306336.1
(22) Date of filing: 11.08.1999
(51) Int. Cl.: C07D 319/08, A01N 43/16

(54) **Benzodioxincarboxylic acid hydrazides as insecticides**

(30) Priority: 25.08.1998 US 97855 P
(71) Applicant: ROHM AND HAAS COMPANY, Philadelphia, Pennsylvania 19106-2399 (US)
(72) Inventor: Opie, Thomas Ranson, North Wales, PA 19454 (US)
(74) Representative: Davis, Carole Amanda

(57) **Abstract**

The present invention is concerned with benzodioxincarboxylic acids and esters, N'-substituted N,N'-diacylhydrazines prepared from benzodioxincarboxylic acids and esters, insecticidal compositions containing the N'-substituted N,N'-diacylhydrazines, and the use of the N'-substituted-N,N'-diacylhydrazines as insecticides.

## Description

The present invention is concerned with benzodioxincarboxylic acids and esters, N'-substituted N,N'-diacylhydrazines prepared from benzodioxincarboxylic acids and esters, insecticidal compositions containing the N'-substituted N,N'-diacylhydrazines, and the use of the N'-substituted-N,N'-diacylhydrazines as insecticides.

The search for compounds which have excellent insecticidal activity is a continuing search because of factors such as the desire for compounds exhibiting greater activity, better selectivity, lower undesirable environmental impact, lower production and market cost and higher effectiveness against insects which are or become resistant to many known insecticides.

There, therefore, continues to be a need to develop further insecticidal compounds having good insecticidal activity. The present invention provides N'-substituted N,N'-diacylhydrazines having good insecticidal properties, 1,3-benzodioxincarboxylic acids useful in the preparation of the N'-substituted N,N'-diacylhydrazines, and a method for preparing such 1,3-benzodioxincarboxylic acids.

Benzo-1,3-dioxins and N'-substituted-N,N'-diacylhydrazines are known in the art and the latter are known to be useful as insecticides. For example, Alain Denis, Michel Delmas and Antoine Gaset, J. Heterocycl. Chem., 1984, 21(2), pages 517-519 disclose the preparation of certain 4-*H*-benzo-1,3 dioxins including 4-*H*-1,3-benzodioxin-6-carboxylic acid, and H. Kämmerer and W. Lotz, Monatsh. Chem., 1971, 102(4), pages 946-950 disclose the preparation of 4-*H*-1,3-benzodioxin-6-carboxylic acid and esters and also certain 8-halo-substituted benzo-1,3-dioxin carboxylic acids and esters. Further, Japanese Patent Application No. 03-141245 discloses a very general formula for N'-t-butyl-N,N'-diacylhydrazines and their use as insecticides. However, no N-(4-*H*-1,3-benzodioxincarbonyl)-N'-t-butyl-N'-acylhydrazines are specifically disclosed in Japanese Patent Application No. 03-141245.

None of the above documents disclose the specific benzo-1,3-dioxin carboxylic acids, or N'-substituted-N,N'-diacylhydrazines with which the present invention is concerned.

According to the present invention there is provided a 4-*H*-1,3-benzodioxincarboxylic acid or ester having the structural formula (I): 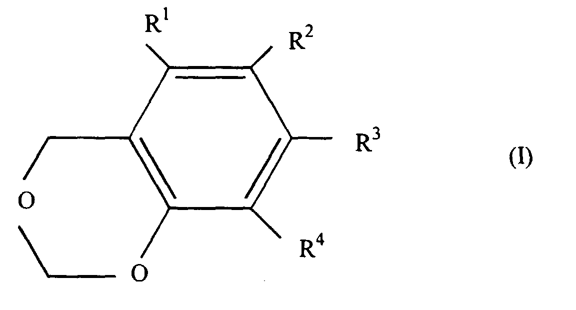 wherein:
one of R¹, R² and R³ is -COOR⁵ wherein R⁵ is selected from hydrogen, alkyl, and haloalkyl, and R⁴ and the others of R¹, R² and R³ are independently selected from hydrogen, alkyl, halo, haloalkyl and alkoxy; and
provided that: (a) at least one of R¹, R², R³ and R⁴ is hydrogen; (b) the 4-*H*-1,3-benzodioxincarboxylic acid or ester is not an unsubstituted 4-*H*-1,3-benzodioxin-6-carboxylic acid or ester, and (c) when R¹ and R³ are hydrogen, and R² is -COOR⁵, then R⁴ is not halo.

As used herein, and unless specified otherwise, "alkyl" refers to an alkyl group containing from 1 to 20 carbon atoms, preferably from 1 to 4 carbon atoms, "haloalkyl" refers to a haloalkyl group containing from 1 to 20 carbon atoms, preferably from 1 to 4 carbon atoms, and "alkoxy" refers to an alkoxy group containing from 1 to 20 carbon atoms, preferably from 1 to 4 carbon atoms.

When R⁴ is halo, then preferably R² is -COOR⁵.

In one embodiment, when R² is -COOR⁵, and R¹ and R³ are independently selected from hydrogen, alkyl, halo, haloalkyl and alkoxy, then R⁴ is not halo.

In one embodiment, (i) R¹ and R² are hydrogen; (ii) R³ is -COOH; and (iii) R⁴ is selected from hydrogen, (C₁-C₄)alkyl and halo(C₁-C₄)alkyl. Preferably, R⁴ is selected from methyl and ethyl.

In another embodiment (i) R¹ and R³ are independently selected from (C₁-C₄)alkyl, halo, halo(C₁-C₄)alkyl, and (C₁-C₄)alkoxy; (ii) R² is -COOH; and (iii) R⁴ is hydrogen.

In a further embodiment of the present invention:
(a). one of R¹ and R³ is -COOR⁵ wherein R⁵ is selected from hydrogen, alkyl, and haloalkyl, and R² and R⁴ and the other of R¹ and R³ are independently selected from hydrogen, alkyl, halo, haloalkyl and alkoxy;
   provided that at least one of R¹, R², R³ and R⁴ is hydrogen; or
(b). R² is -COOR⁵ wherein R⁵ is selected from hydrogen, alkyl, and haloalkyl;
   R¹ and R³ are independently selected from hydrogen, alkyl, halo, haloalkyl and alkoxy; and
   R⁴ is selected from hydrogen, alkyl, haloalkyl and alkoxy;
   provided that at least one but not all of R¹, R³ and R⁴ is hydrogen.

Preferably, in this further embodiment, one of R¹ and R³ is -COOR⁵ wherein R⁵ is selected from hydrogen, alkyl, and haloalkyl, and R² and R⁴ and the other of R¹ and R³ are independently selected from hydrogen, alkyl, halo, haloalkyl and alkoxy, provided that at least one of R¹, R², R³ and R⁴ is hydrogen.

More preferably, in this further embodiment (i) R¹ and R² are hydrogen; (ii) R³ is -COOH; and (iii) R⁴ is selected from hydrogen, (C₁-C₄)alkyl, and halo(C₁-C₄)alkyl. Even more preferably, R⁴ is selected from methyl and ethyl.

Alternatively, in the above further embodiment, (i) one of R¹ and R³ is selected from (C₁-C₄)alkyl, halo, halo(C₁-C₄)alkyl, and (C₁-C₄)alkoxy; (ii) R² is -COOH; and (iii) R⁴ and the other of R¹ and R³ is hydrogen.

The present invention also provides a process for preparing the above 4-*H*-1,3-benzodioxincarboxylic acids and esters, which comprises reacting a hydroxybenzoic acid or ester having the structural formula (II): 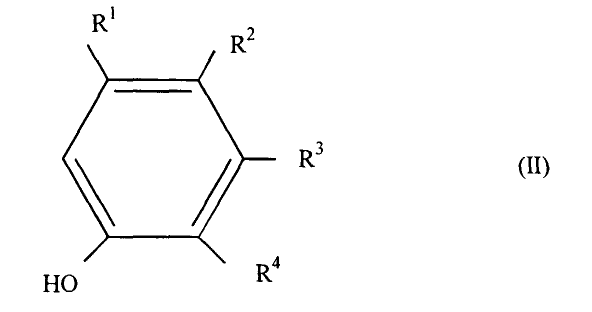 wherein R¹, R², R³, and R⁴ are as defined above,
with substantially dry formaldehyde gas or substantially dry formaldehyde-generating compound, preferably paraformaldehyde or 1,3,5-trioxane, in the presence of a Lewis acid or Bronsted acid catalyst and, optionally, in the presence of a non-reactive organic solvent, and wherein the level of water during the reaction (i.e. any water present as a result of the reactants used plus any water produced during the reaction) does not exceed 30 weight percent, and preferably does not exceed 15 weight percent, based on the weight of the hydroxybenzoic acid or ester.

In the above method, when the 4-*H*-1,3-benzodioxincarboxylic acid or ester produced is an ester in which R⁵ is selected from alkyl, and haloalkyl, the ester may be hydrolysed with a strong base in the presence of water or a wet organic solvent to produce the corresponding 4-*H*-1,3-benzodioxincarboxylic acid. The organic solvent is preferably miscible with water (e.g. methanol or ethanol).

The Lewis acid or Bronsted acid catalyst may be any strong Bronsted acid or strong Lewis acid, for example, para-toluenesulfonic acid, methanesulfonic acid, sulfuric acid, phosphoric acid, hydrochloric acid, sulfamic acid, the acid forms of ion exchange resins (e.g. Amberlyst 15, "Amberlyst" is a trademark of Rohm and Haas Company), scandium salts of strong acids such as scandium (III) trifluoromethanesulfonate and other scandium salts of strong acids such as methanesulfonate or p-toluenesulfonate, lanthanide trifluoromethanesulfonates such as ytterbium (III) trifluoromethane-sulfonate, montmorillonite clay, boron trifluoride etherate, BF₃, PF₅, Ti(OiPr)₄ (titanium tetraisopropoxide), and AlCl₃. Preferably, the acid catalyst does not contain chlorine or bromine. More preferably, the acid catalyst is selected from para-toluenesulfonic acid, methanesulfonic acid, sulfuric acid, phosphoric acid, sulfamic acid, the acid forms of ion exchange resins, scandium salts of strong acids, lanthanide trifluoromethanesulfonates, and montmorillonite clay. More preferably, the acid catalyst is para-toluenesulfonic acid or methanesulfonic acid.

The acid catalyst is preferably present in an amount of 0.1 to 20 wt. %, more preferably 1 to 5 wt. %, based on the weight of the hydroxybenzoic acid or ester of formula (II). Preferably, the acid catalyst does not contribute more than 0.1 weight percent water, based on the weight of the hydroxybenzoic acid, to the reaction. Preferably, the acid catalyst contains no more than 10 weight percent water.

Any organic solvent that does not react with the acid catalyst and the formaldehyde gas or formaldehyde-generating compound may be used in the above method. Suitable non-reactive organic solvents include, for example, dichloroethane, trichloroethylene, sulfolane, toluene, ortho- or meta-dichlorobenzene, and chlorobenzene. Preferably, the non-reactive organic solvent is toluene, chlorobenzene or ortho- or meta-dichlorobenzene.

Preferably, the temperature in the above process is from 0°C to 180°C, more preferably from 40°C to 160°C. Preferably, the time of reaction is from 15 seconds to 24 hours.

The hydroxybenzoic acids and esters of formula (II), used as starting materials in the above described method, may, for example, be prepared by known methods.

A preferred method for preparing the hydroxybenzoic acids of formula (II), comprises reacting the corresponding alkoxybenzoic acid with a hydrohalic acid in a suitable solvent, for example, with HBr and glacial acetic acid. This may be represented by the following reaction for the preparation of 2-methyl-3-hydroxybenzoic acid from 2-methyl-3-methoxy benzoic acid:

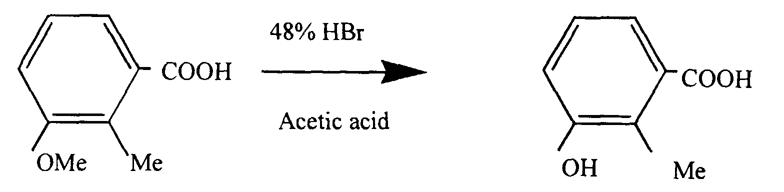

The hydroxybenzoic acid esters of formula (II) may, for example, be prepared from the corresponding benzoic acids by reacting the acid with a reagent capable of forming an activated carboxylic acid (e.g. 2-chloro-4,6-dimethoxy-1,3,5-triazine, POCl₃, oxalyl chloride, DCC or sulfur oxyhalide such as thionyl chloride) and the appropriate alcohol. For example, 2-methyl-3-hydroxy benzoic acid methyl ester may be prepared according to the following reaction:

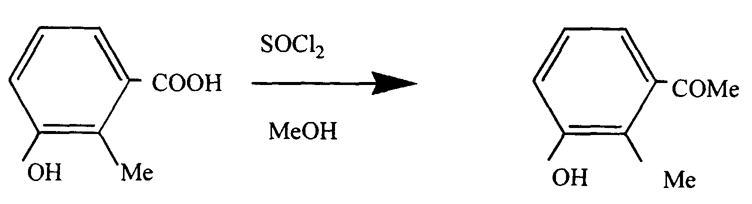

The present invention further provides an N'-substituted-N,N'-diacylhydrazine having the structural formula (III): 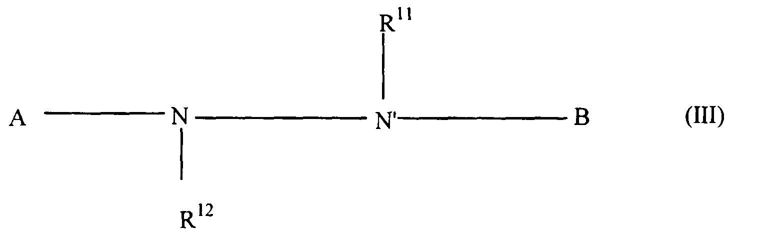 wherein:
(i). one of R¹¹ and R¹² is hydrogen and the other of R¹¹ and R¹² is unsubstituted (C₃-C₁₀)branched alkyl or a (C₁-C₄)straight chain alkyl substituted with one or two of the same or different (C₃-C₆)cycloalkyl;
(ii). A is: 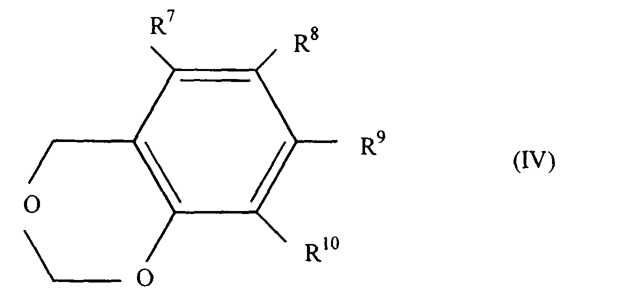 wherein:
   one of R⁷, R⁸ or R⁹ is a -C(O)- group, and R¹⁰ and the others of R⁷, R⁸ and R⁹ are independently selected from hydrogen, alkyl, halo, haloalkyl and alkoxy;
   provided that at least one of R⁷, R⁸, R⁹ and R¹⁰ is hydrogen; and
(iii) B is -C(O)-Z
   wherein Z is:
   phenyl;
   naphthyl; or
   phenyl or naphthyl substituted with one to three of the same or different substituents selected from the group consisting of: halo; cyano; nitro; hydroxy; mercapto; thiocyanato; (C₁-C₄)alkyl; (C₁-C₄)alkoxy; halo(C₁-C₂)alkyl; halo(C₁-C₂)alkoxy; (C₁-C₄)alkylthio; (C₁-C₄)alkylsulfinyl; (C₁-C₄)alkylsulfonyl; carboxy; formyl; (C₁-C₄)alkylcarbonyl; (C₁-C₄)alkoxycarbonyl; (C₁-C₄)alkanoyloxy; amino; (C₁-C₄)alkylamino; di(C₁-C₄)alkylamino having independently the stated number of carbon atoms in each alkyl group; carbamoyl; (C₁-C₄)alkylcarbamoyl; di(C₁-C₄)alkylcarbamoyl having independently the stated number of carbon atoms in each alkyl group; cyano(C₁-C₄)alkyl; (C₁-C₄)alkoxy(C₁-C₄)alkyl; (C₂-C₆)alkenyl; (C₄-C₆)alkadienyl; (C₂-C₆)alkynyl; (C₁-C₄)alkyldithionate; (C₁-C₄)alkylcarbonylthio; tri(C₁-C₄)alkylsilyl having independently the stated number of carbon atoms in each alkyl group; phenyl; phenyl substituted with one to two of the same or different substituents selected from the group consisting of halo, cyano, nitro, hydroxy, (C₁-C₄)alkyl, (C₁-C₄)alkoxy, halo(C₁-C₂)alkyl, halo(C₁-C₂)alkoxy, (C₁-C₄)alkylthio, (C₁-C₄)alkylsulfinyl, (C₁-C₄)alkylsulfonyl, carboxy, formyl, (C₁-C₄)alkylcarbonyl, (C₁-C₄)alkoxycarbonyl, (C₁-C₄)alkanoyloxy, amino, (C₁-C₄)alkylamino, di(C₁-C₄)alkylamino having independently the stated number of carbon atoms in each alkyl group; phenoxy; phenoxy substituted with one to two of the same or different substituents selected from the group consisting of halo, cyano, nitro, hydroxy, (C₁-C₄)alkyl, (C₁-C₄)alkoxy, halo(C₁-C₂)alkyl, halo(C₁-C₂)alkoxy, (C₁-C₄)alkylthio, (C₁-C₄)alkylsulfinyl, (C₁-C₄)alkylsulfonyl, carboxy, formyl, (C₁-C₄)alkylcarbonyl, (C₁-C₄)alkoxycarbonyl, (C₁-C₄)alkanoyloxy, amino, (C₁-C₄)alkylamino, di(C₁-C₄)alkylamino having independently the stated number of carbon atoms in each alkyl group; benzoyl; benzoyl substituted with one to two of the same or different substituents selected from the group consisting of halo, cyano, nitro, hydroxy, (C₁-C₄)alkyl, (C₁-C₄)alkoxy, halo(C₁-C₂)alkyl, halo(C₁-C₂)alkoxy, (C₁-C₄)alkylthio, (C₁-C₄)alkylsulfinyl, (C₁-C₄)alkylsulfonyl, carboxy, formyl, (C₁-C₄)alkylcarbonyl, (C₁-C₄)alkoxycarbonyl, (C₁-C₄)alkanoyloxy, amino, (C₁-C₄)alkylamino, di(C₁-C₄)alkylamino having independently the stated number of carbon atoms in each alkyl group; phenoxycarbonyl; phenoxycarbonyl substituted with one to two of the same or different substituents selected from the group consisting of halo, cyano, nitro, hydroxy, (C₁-C₄)alkyl, (C₁-C₄)alkoxy, halo(C₁-C₂)alkyl, halo(C₁-C₂)alkoxy, (C₁-C₄)alkylthio, (C₁-C₄)alkylsulfinyl, (C₁-C₄)alkylsulfonyl, carboxy, formyl, (C₁-C₄)alkylcarbonyl, (C₁-C₄)alkoxycarbonyl, (C₁-C₄)alkanoyloxy, amino, (C₁-C₄)alkylamino, di(C₁-C₄)alkylamino having independently the stated number of carbon atoms in each alkyl group; phenylthio; phenylthio substituted with one to two of the same or different substituents selected from the group consisting of halo, cyano, nitro, hydroxy, (C₁-C₄)alkyl, (C₁-C₄)alkoxy, halo(C₁-C₂)alkyl, halo(C₁-C₂)alkoxy, (C₁-C₄)alkylthio, (C₁-C₄)alkylsulfinyl, (C₁-C₄)alkylsulfonyl, carboxy, formyl, (C₁-C₄)alkylcarbonyl, (C₁-C₄)alkoxycarbonyl, (C₁-C₄)alkanoyloxy, amino, (C₁-C₄)alkylamino, di(C₁-C₄)alkylamino having independently the stated number of carbon atoms in each alkyl group; phenyl(C₁-C₄)alkyl; phenyl(C₁-C₄)alkyl substituted on the phenyl ring with one to two of the same or different substituents selected from the group consisting of halo, cyano, nitro, hydroxy, (C₁-C₄)alkyl, (C₁-C₄)alkoxy, halo(C₁-C₂)alkyl, halo(C₁-C₂)alkoxy, (C₁-C₄)alkylthio, (C₁-C₄)alkylsulfinyl, (C₁-C₄)alkylsulfonyl, carboxy, formyl, (C₁-C₄)alkylcarbonyl, (C₁-C₄)alkoxycarbonyl, (C₁-C₄)alkanoyloxy, amino, (C₁-C₄)alkylamino, di(C₁-C₄)alkylamino having independently the stated number of carbon atoms in each alkyl group; or when two adjacent positions on a phenyl ring are substituted with alkoxy groups, these groups may be joined to form a 5 or 6 membered dioxolano (methylenedioxy) or dioxano (1,2-ethylenedioxy) heterocyclic ring.

Preferably, R¹² is hydrogen.

Preferably, R¹¹ is t-butyl.

Preferably, when R⁸ is -C(O)-, then R⁷, R⁹ and R¹⁰ are not all hydrogen.

In one embodiment of the N'-substituted-N,N'-diacylhydrazines according to the present invention, (i) R⁷ and R⁸ are hydrogen; (ii) R⁹ is -C(O)-; and (iii) R¹⁰ is selected from hydrogen, (C₁-C₄)alkyl and halo(C₁-C₄)alkyl. Even more preferably, R¹⁰ is selected from methyl and ethyl.

In another embodiment of the N'-substituted-N,N'-diacylhydrazines of the present invention, (i) R⁷ and R⁹ are independently selected from (C₁-C₄)alkyl, halo, halo(C₁-C₄)alkyl, and (C₁-C₄)alkoxy; (ii) R⁸ is -C(O)-; and (iii) R¹⁰ is hydrogen.

In a further embodiment of the N'-substituted-N,N'-diacylhydrazines according to the present invention:
(a) one of R⁷ and R⁹ is -C(O)-, and R⁸ and R¹⁰ and the other of R⁷ and R⁹ are independently selected from hydrogen, alkyl, halo, haloalkyl and alkoxy;
   provided that at least one of R⁷, R⁸, R⁹ and R¹⁰ is hydrogen; or
(b). R⁸ is -C(O)-;
   R⁷ and R⁹ are independently selected from hydrogen, alkyl, halo, haloalkyl and alkoxy; and
   R¹⁰ is selected from hydrogen, alkyl, haloalkyl and alkoxy;
   provided that at least one but not all of R⁷, R⁹ and R¹⁰ is hydrogen.

Preferably, in the above further embodiment of the N'-substituted-N,N'-diacylhydrazines according to the present invention, one of R⁷ and R⁹ is -C(O)-, and R⁸ and R¹⁰ and the other of R⁷ and R⁹ are independently selected from hydrogen, alkyl, halo, haloalkyl and alkoxy; provided that at least one of R⁷, R⁸, R⁹ and R¹⁰ is hydrogen.

More preferably, in the above further embodiment of the N'-substituted-N,N'-diacylhydrazines according to the present invention, (i) R⁷ and R⁸ are hydrogen; (ii) R⁹ is -C(O)-; and (iii) R¹⁰ is selected from hydrogen (C₁-C₄)alkyl and halo(C₁-C₄)alkyl. Even more preferably, R¹⁰ is selected from methyl and ethyl.

Alternatively, in the above further embodiment of the N'-substituted-N,N'-diacylhydrazines according to the present invention, (i) R⁷ and R⁹ are independently selected from (C₁-C₄)alkyl, halo, halo(C₁-C₄)alkyl, and (C₁-C₄)alkoxy; (ii) R⁸ is -C(O)-; and (iii) R¹⁰ is hydrogen.

The N'-substituted-N,N'-diacylhydrazines of the present invention may, for example, be prepared by methods similar to those disclosed in EP-B-0 236 618.

For example, the N'-substituted-N,N'-diacylhydrazines of the present invention may be prepared by a process, which comprises:
(a). reacting a 4-*H*-1,3-benzodioxincarboxylic acid or ester having the structural formula (I) defined above except that (a) the compound of formula (I) may also be an unsubstituted 4-*H*-1,3-benzodioxin-6-carboxylic acid or ester, and (b) when R¹ and R³ are hydrogen, and R² is -COOR⁵, then R⁴ may also be halo, with oxalyl chloride or a phosphorus or sulfur halide or oxyhalide, optionally in the presence of an N,N-dialkylcarboxamide catalyst and/or a non-reactive organic solvent, to produce the corresponding 4-*H*-1,3-benzodioxincarboxylic acid chloride; and
(b). reacting the corresponding 4-*H*-1,3-benzodioxincarboxylic acid chloride with a compound having the structural formula: 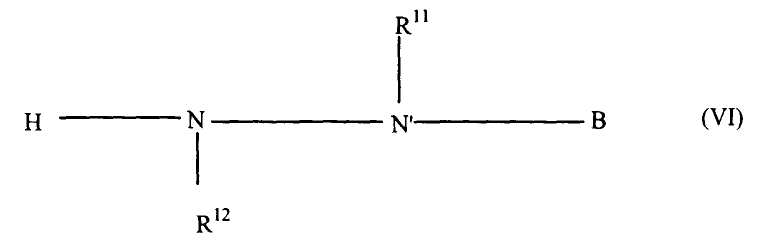 wherein B, R¹¹ and R¹² are as defined above,
at a temperature of -50 to 120°C in the presence of an organic or inorganic base, and optionally an organic solvent and/or water, to produce the compound of structural formula (III).

Solvents which may be used in step (a) include, for example, hydrocarbons such as toluene and benzene, ethers such as diethyl ether and glyme, haloalkanes such as methylene chloride, dimethylformamide, and mixtures of such solvents.

Solvents which may be used in step (b) include, for example, hydrocarbons such as toluene, xylene, hexane and heptane, esters such as propylacetate, ethers such as tetrahydrofuran and glyme, acetonitrile, pyridine, haloalkanes such as methylene chloride, and mixtures of such solvents.

An alternative method for preparing the N'-substituted-N,N'-diacylhydrazines of the present invention comprises:
(a). reacting a 4-*H*-1,3-benzodioxincarboxylic acid or ester having the structural formula (I) defined above except that (a) the compound of formula (I) may also be an unsubstituted 4-*H*-1,3-benzodioxin-6-carboxylic acid or ester, and (b) when R¹ and R³ are hydrogen, and R² is -COOR⁵, then R⁴ may also be halo, with oxalyl chloride or a phosphorus or sulfur halide or oxyhalide, optionally in the presence of a N,N-dialkylcarboxamide catalyst and/or a non-reactive organic solvent, to produce the corresponding 4-*H*-1,3-benzodioxincarboxylic acid chloride;
(b). reacting the 4-*H*-1,3-benzodioxincarboxylic acid chloride, optionally in the presence of an organic solvent, with a hydrazine of formula:

   (R¹¹)NH-NH(R¹²)

   wherein R¹¹ and R¹² are as defined above, to produce a compound having the structural formula: 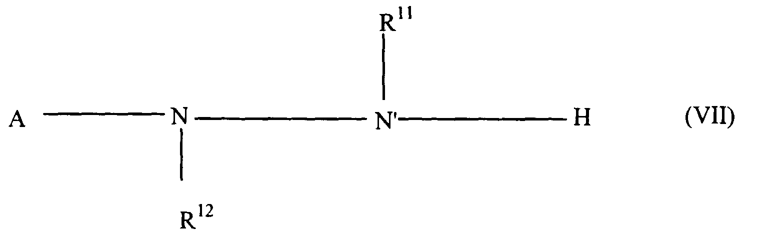 wherein A, R¹¹ and R¹² are as defined above; and
(c). reacting the compound of formula VII with an acid chloride having the structural formula: 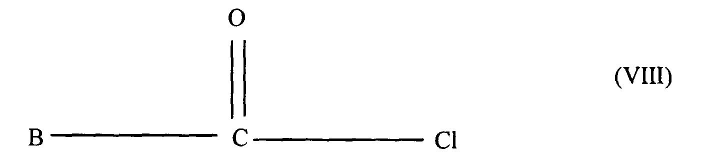 wherein B is as defined above,
at a temperature of -50 to 120°C in the presence of an organic or inorganic base, and optionally an organic solvent and/or water, to produce the compound of structural formula (III).

Suitable non-reactive organic solvents, which may be used in step (a) of the above alternative method for preparing the N'-substituted-N,N'-diacylhydrazines of the present invention, include, for example, hydrocarbons such as toluene and benzene, ethers such as diethyl ether and glyme, haloalkanes such as methylene chloride, dimethylformamide, and mixtures of such solvents.

Suitable solvents, which may be used steps (b) and (c) of the above alternative method for preparing the N'-substituted-N,N'-diacylhydrazines of the present invention, include, for example, hydrocarbons such as toluene, xylene, hexane and heptane, esters such as propyl acetate, ethers such as tetrahydrofuran and glyme, acetonitrile, pyridine, haloalkanes such as methylene chloride, and mixtures of such solvents.

A further alternative method for preparing the N'-substituted-N,N'-diacylhydrazines of the present invention comprises:
(a). reacting a 4-*H*-1,3-benzodioxincarboxylic acid ester having the structural formula (I) defined above except that (a) the compound of formula (I) may also be an unsubstituted 4-*H*-1,3-benzodioxin-6-carboxylic ester, and (b) when R¹ and R³ are hydrogen, and R² is -COOR⁵, then R⁴ may also be halo, with a compound having the structural formula (VI): 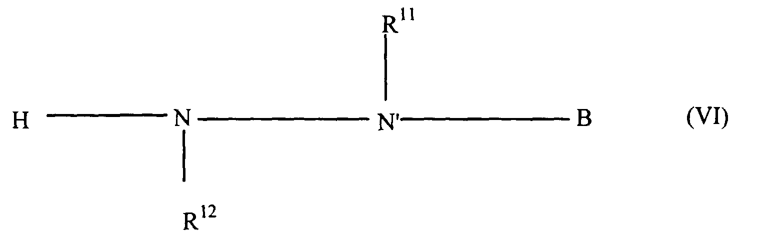 wherein B, R¹¹ and R¹² are as defined above,
at a temperature of -50 to 150°C, to produce the compound of structural formula (III).

In this further alternative method for preparing the N'-substituted-N,N'-diacylhydrazines of the present invention, the reaction may, for example, be conducted in the presence of an organic or inorganic base.

In this further alternative method for preparing the N'-substituted-N,N'-diacylhydrazines of the present invention, the reaction may, for example, be conducted in the presence of an organic solvent and/or water.

In this further alternative method for preparing the N'-substituted-N,N'-diacylhydrazines of the present invention, the temperature is preferably from 100°C to 150°C.

Organic solvents, which may be used in the above further alternative method for preparing the N'-substituted-N,N'-diacylhydrazines of the present invention, include, for example, hydrocarbons such as toluene, xylene, hexane and heptane, alcohols such as methanol, ethanol and isopropanol, ethers such as tetrahydrofuran and glyme, acetonitrile, pyridine, haloalkanes such as methylene chloride, and mixtures of such solvents.

Organic and inorganic bases, which may be used in the above methods for preparing the N'-substituted-N,N'-diacylhydrazines of the present invention, include, for example, tertiary amines such as triethylamine, pyridine, potassium carbonate, sodium carbonate, sodium bicarbonate, sodium hydroxide and potassium hydroxide. Preferably, the base is selected from sodium hydroxide, potassium hydroxide and triethylamine.

The N'-substituted-N,N'-diacylhdrazines according to the present invention are effective as insecticides. They may, for example, be used to control insects of the order Lepidoptera, Coleoptera, and Homoptera, especially insects of the order Lepidoptera. Examples, of Lepidoptera that may be controlled by the N'-substituted-N,N'-diacylhydrazines according to the invention, include Southern armyworm (*Spodoptera eridania*), Tobacco budworm (*Heliothis virescens*), Diamondback moth, Cabbage looper, and Beet armyworm.

According to the present invention there is further provided an insecticidal composition, which comprises an N'-substituted-N,N'-diacylhydrazine according to the invention and an agronomically acceptable carrier. The agronomically acceptable carrier may, for example, be a conventional inert (i.e. plant compatible) diluent or extender such as solid carrier material or liquid carrier material of the type usable in conventional agricultural compositions or formulations. If desired, adjuvants such as surfactants, stabilizers, antifoam agents, and antidrift agents may be employed in the insecticidal compositions of the present invention. Examples of suitable compositions are aqueous solutions and dispersions, dusting powders, granules, oily solutions, oil dispersions, pastes, wettable powders, emulsifiable concentrates, flowables, baits, invert emulsions, aerosol compositions and fumigating candles. The compositions may be prepared in a known manner, for example, by extending the N'-substituted-N,N'-diacylhydrazines with conventional dispersible liquid diluent carriers and/or dispersible solid carriers optionally with the use of carrier vehicle assistants, e.g. conventional surface-active agents, including emulsifying agents and/or dispersing agents.

An N'-substituted-N,N'-diacylhydrazine according to the present invention may be used alone, or in the form of a mixture of two or more such N'-substituted-N,N'-diacylhydrazines. However, for practical reasons, the N'-substituted-N,N'-diacylhydrazine(s) are generally used in the form of compositions containing an agronomically acceptable carrier and, optionally, one or more of the adjuvants as described above.

In the insecticidal compositions according to the present invention the N'-substituted-N,N'-diacylhydrazine is generally present in an amount of from about 0.0001 to about 99 percent by weight, and preferably in an amount of from about 1 to 75 percent by weight, based on the weight of the composition.

The present invention still further provides a method of controlling insects, which comprises applying to a plant, to the locus of a plant, or to seeds, an N'-substituted-N,N'-diacylhydrazine according to the invention or a composition according to the invention. Preferably, the N'-substituted-N,N'-diacylhydrazine according to the invention, or a composition according to the invention, is applied to a plant or to the locus of a plant.

The N'-substituted-N,N'-diacylhydrazines according to the invention, and the compositions according to the invention, may, for example, be applied to the plants or to the locus of the plants by spray methods commonly employed, such as conventional high gallonage hydraulic sprays, low gallonage sprays, ultra-low-volume sprays, airblast sprays, aerial sprays and dusts as is known in the art. The N'-substituted-N,N'-diacylhydrazines according to the invention, and the compositions according to the invention, may also be strewn mechanically or by hand as dusts or granules.

In general, for the control of insects in agriculture, horticulture and forestry, the N'-substituted-N',N-diacylhydrazine compounds of the present invention may be used at a dosage corresponding to from about 10 grams to about 10 kilograms, preferably from about 100 grams to about 5 kilograms, of the compound per hectare. The exact amount of dosage for a given situation can be routinely determined and depends on a variety of factors, for example, the particular substance used, the kind of insect, the formulation/composition used, the state of the crop infested with the insect and the prevailing weather conditions. The term "insecticidal", as employed in the present application, is to be construed as any means which adversely affects the existence or growth of the target insects at any stage in their life cycle. Such means can comprise a complete killing action, eradication, arresting in growth, inhibition, reducing in number, reproductive inhibition, or any combination thereof.

In the above method for controlling insects, the term "locus of a plant" is intended to mean an area where a crop is growing and an area where a crop is to be grown.

The N'-substituted-N,N'-diacylhydrazines according to the invention, and the compositions according to the invention, may also be useful in controlling insects in seeds, for example, by applying the N'-substituted-N,N'-diacylhydrazine or the composition to the surface area of the seeds to be treated. This may, for example, be accomplished by varying means common in the art, such as slurrying, soaking, dusting, and spraying.

The N'-substituted-N,N'-diacylhydrazines according to the invention may, for example, be used in combination with other known pesticidal compounds. This may lead to an expansion in the spectrum of activity of the preparations.

Some embodiments of the present invention will now be described in detail in the following Examples.

### Examples

### Example 1.

### Preparation of 8-methyl-4-H-1,3-benzodioxin-7-carboxylic acid

This Example illustrates the preparation of 8-methyl-4-*H*-1,3-benzodioxin-7-carboxylic acid from methyl 2-methyl-3-hydroxybenzoate. It also illustrates the preparation of such methyl 2-methyl-3-hydroxybenzoate from the corresponding hydroxybenzoic acid, and the preparation of the hydroxybenzoic acid from the corresponding methoxybenzoic acid.

### (i). Preparation of 2-methyl-3-hydroxybenzoic acid:

2-methyl-3-methoxybenzoic acid (8.00 g,48 mmole), glacial acetic acid (17.9 g) and 48.5 wt. % aqueous hydrobromic acid (20.1 g, 121 meq) were heated in a 125 ml erlenmeyer flask for 9.3 hours at 104-122°C in an oil bath. After 2.2 hours, additional 48.5% hydrobromic acid (8.89 g, 53.3 meq) was added. At the end of the reaction, the mixture was neutralized with saturated potassium carbonate solution (33.4 g), diluted with 14.5 g deionized water and extracted with several portions of diethyl ether with acidifications to pH 1 with 37% hydrochloric acid as required. The combined extracts were evaporated using a Rotovap evaporator and blown down with heating to 8.02 g of brown oil with an acetic acid odor. Addition of 7.95 g toluene and evaporation gave crude 2-methyl-3-hydroxybenzoic acid as a tan solid, m.p. 126-140°C, mostly 136-140°C (7.63 g, ca. 5% 2-methyl-3-methoxybenzoic acid by NMR).

### (ii) Preparation of methyl 2-methyl-3-hydroxybenzoate.

Crude 2-methyl-3-hydroxybenzoic acid (7.63 g) was dissolved in methanol (18.45 g) in a 300 ml round bottom flask and 99% thionyl chloride (9.8 g, 82 mmole) was added dropwise with stirring. The resulting homogeneous solution was heated in an oil bath for 10 minutes at 46-70°C and 65 minutes at 70-85°C bath temperature. The slowly refluxing mixture was evaporated to 8.22 g of brown oil, 1.81 g of toluene was added and the mixture was evaporated again to give methyl 2-methyl-3-hydroxybenzoate (8.15 g) as a brown solid, m.p. 66.8-73.2°C.

### (iii). Preparation of 8-methyl-4-H-1,3,-benzodioxin-7-carboxylic acid

p-Toluenesulfonic acid monohydrate catalyst (0.82 g, 4.3 mmole) was dried by melting in 1.34 g xylenes, evaporating the hot liquid mixture, melting with 0.84 g xylenes and evaporating the hot mixture to 0.74 g. This was transferred to the 300 ml round bottom flask containing crude methyl 2-methyl-3-hydroxybenzoate (8.15 g) with several hot toluene washes and paraformaldehyde (6.14 g, 204 mmoles as formaldehyde monomer) and toluene (total 63 g) were added. The stirred slurry was heated for 19 minutes at 97-121°C bath temperature and 89 minutes at 121-126°C bath temperature (slow reflux). GC (gas chromatographic) analysis indicated starting ester was almost totally absent. The mixture was cooled and the toluene supernate, two toluene washes (17 g, 4.1 g) and one methanol wash (15 g) of the paraformaldehyde solid were filtered through glass wool, combined in a 300 ml round bottom flask and evaporated to 6.63 g of oil and solid. This was dissolved in 17.3 g methanol and 50% aqueous sodium hydroxide (5.22 g, 65.6 meq) was added. The solution was heated with stirring for 10 minutes at 65-69°C bath temperature. Then 6.08 g deionized water was added and heating was continued for 13 minutes. The homogeneous solution was evaporated to 24.4 g, 3.31 g deionized water was added and refluxing was continued for 20 minutes. Then 3.30 g solvent was evaporated and the turbid mixture was diluted with 10.76 g deionized water and extracted several times with diethyl ether to remove non-acidic impurities. Acidification with 37% aqueous hydrochloric acid, filtration of the chilled mixture with deionized water washes, and drying gave crude 8-methyl-4-*H*-1,3-benzodioxin-7-carboxylic acid (5.35 g, 57% overall yield) as a tan solid, m.p. 149-174°C, mostly 159-174°C. The GC purity of the crude product was 86.5 area %. The crude solid was purified by partitioning between 27.8 g propyl acetate and 26.4 g deionized water containing 1.98 g or 24.9 meq of 50% aqueous sodium hydroxide to give, after acidification and filtration of the aqueous layer, purified 8-methyl-4-*H*-1,3-benzodioxin-7-carboxylic acid (4.39 g, 46% overall yield), m.p. 167-177°C.

### Example 2.

### Preparation of 8-ethyl-4-H-1,3-benzodioxin-7-carboxylic acid

This Example illustrates the preparation of 8-ethyl-4-*H*-1,3-benzodioxin-7-carboxylic acid from methyl 2-ethyl-3-hydroxybenzoate. It also illustrates the preparation of such methyl 2-ethyl-3-hydroxybenzoate from the corresponding hydroxybenzoic acid, and the preparation of the hydroxybenzoic acid from the corresponding methoxybenzoic acid.

### (i). Preparation of 2-ethyl-3-methoxybenzoic acid and methyl-2-ethyl-3-hydroxybenzoate.

By a similar procedure to that used in Example 1 above, 2-ethyl-3-methoxybenzoic acid (3.95 g, 21.9 mmole) was demethylated to give crude 2-ethyl-3-hydroxybenzoic acid (4.30 g, acetic acid odor, m.p. after drying 137-162°C, mostly 156-162°C). The crude acid was converted to crude methyl-2-ethyl-3-hydroxybenzoate (4.11 g, m.p. 79-96°C) by adding thionyl chloride to a methanol solution of the acid and heating as in Example 1.

### (ii). Preparation of 8-ethyl-4-H-1,3-benzodioxin-7-carboxylic acid.

Crude methyl 2-ethyl-3-hydroxybenzoate (4.11 g, <21.9 mmole), paraformaldehyde (2.89 g or 96 mmole as formaldehyde monomer), 0.58 g xylenes-dried p-toluenesulfonic acid (originally 0.64 g monohydrate), and 23.6 g toluene were heated with stirring in a 300 ml round bottom flask for 84 minutes in a 124-126°C oil bath. Starting ester appeared to be absent after only 61 minutes. The cooled supernate and three diethyl ether washes of the paraformaldehyde solid were evaporated in a 300 ml round bottom flask using a Rotovap evaporator and blown down to 4.59 g of oil and solid. This was dissolved in 8.02 g methanol and 50% aqueous sodium hydroxide (3.91 g, 49 meq) was added. The solution was heated for 22 minutes at 64°C, 3.17 g deionized water was added, and heating was continued for 22 minutes at 64°C. Then 3.29 g deionized water was added and heating was continued for 31 minutes. The 26.9 g of liquid was evaporated to 20.9 g and partitioned between 6.8 g deionized water and 11.4 g diethyl ether. The ether layer and a 8.8 g ether wash of the aqueous layer were removed and the aqueous layer was partially neutralized with 37% aqueous hydrochloric acid (1.89 g, 19.2 meq). The aqueous layer was extracted with 3.69 g diethyl ether, 0.06 g (37%) hydrochloric acid was added and it was extracted with 3.67 g diethyl ether. Acidification of the final aqueous layer with 37% aqueous hydrochloric acid (2.65 g, 26.9 meq), chilling, filtration and drying gave crude 8-ethyl-4-*H*-1,3-benzodioxin-7-carboxylic acid (3.16 g, 69% crude yield but only 64 area % pure by GC) as a brown solid, m.p. 122-136.5°C. Repeated partitionings of the crude material between less than one equivalent of 50% aqueous sodium hydroxide in water and diethyl ether eventually resulted in purified 8-ethyl-4-*H*-1,3-benzodioxin-7-carboxylic acid (1.90 g, 41.6 % yield) as a tan solid, m.p. 146.8-150.2°C.

### Example 3.

### Preparation of 4-H-1,3-benzodioxin-7-carboxylic acid

This Example illustrates the preparation of *4*-*H*-1,3-benzodioxin-7-carboxylic acid from methyl-3-hydroxybenzoate.

Crude methyl 3-hydroxybenzoate (7.80 g, 51.2 mmole), paraformaldehyde (9.46 g, 315 mmole as formaldehyde monomer), 1.28 g of xylenes-dried p-toluenesulfonic acid catalyst (originally 1.37 g of toluene sulfonic acid monohydrate) and 55.2 g toluene were heated with stirring in a 300 ml round bottom flask for 6.9 hours at 108.5-126.4°C oil bath temperature. The mixture was cooled to room temperature and the supernate and three small toluene washes of insoluble solid (dry wt. 6.91 g) were removed and filtered through glass wool. GC analysis of the combined filtrates gave less than two percent of unreacted starting ester. They were evaporated to 10.25 g of waxy solid, which was suspended in 15.95 g methanol and 50.3 wt. % aqueous sodium hydroxide (8.02 g, 101 meq) was added. Heating for four minutes in a 72°C water bath did not dissolve a pasty mass, so 8.05 g deionized water was added to dissolve most of the solid. After 10 minutes additional heating in the bath at 72°C, 7.62 g deionized water was added, dissolving more of the solid. After 33 minutes additional heating at 72°C, the mixture was cooled and extracted twice with ether/hexanes to remove 0.97 g of non hydrolyzable oil.

Then 6.8 g of solvent was evaporated from the extracted aqueous layer, 18.7 g deionized water was added, and the mixture was acidified to pH1 with 37 % aqueous hydrochloric acid, (14.83 g, 150.5 meq). The resulting white slurry was briefly chilled and filtered with four water washes to give 7.85 g of very crude (40%, 30% and 8 area percent of three largest components) 4-*H*-1,3-benzodioxin-7-carboxylic acid, m.p. 147-186°C. GC/MS (Gas Chromatography/Mass Spectrometry) identified the largest component as the desired 4-*H*-1,3-benzodioxin-7-carboxylic acid and the second largest as the internally cyclized lactone of probable 8-hydroxymethyl-4-*H*-1,3-benzodioxin-7-carboxylic acid. Purified 4-*H*-1,3-benzodioxin-7-carboxylic acid (2.44 g, 26.4% yield, 90-95 area % by GC, m.p. 180-191°C to 192-207°C depending on purity) was eventually isolated from this crude material by a mixture of silica gel column chromatography with ether/hexanes and (more effectively) multiple liquid liquid countercurrent partitions of partially neutralized crude acid fractions between ether and deionized water.

### Example 4.

### Preparation of 8-methoxy-4-H-1,3-benzodioxin-6-carboxylic acid

97% 4-hydroxy-3-methoxybenzoic acid (5.04 g, 30.0 mmoles) (Aldrich Chemical Company), paraformaldehyde (2.86 g, equivalent of 95.2 mmoles formaldehyde monomer), and methanesulfonic acid catalyst (0.17 g, 1.77 mmoles) in 14.30 g o-dichlorobenzene solvent were heated with magnetic stirring in a capped 6 dram vial for 6 hours at 126.2-132.0°C. The initially thick slurry gradually thinned until no solid was present halfway through the reaction. The reaction mixture was cooled and partitioned between 9.23 g deionized water containing 50.0 wt.% aqueous sodium hydroxide (6.22 g, 77.8 meq) and 27.5 g diethyl ether containing 9.0 g dichloromethane. The organic layer was removed and the aqueous layer and a little emulsion were washed three times (11.4 g, 12.7 g. 19.3 g) with diethyl ether. Then the washed aqueous layer was filtered through glass wool and acidifed with 37 wt. % aqueous hydrochloric acid (6.49 g, 65.9 meq) with vigorous shaking. The resulting thick light gray slurry was chilled for 30 minutes in a refrigerator then filtered with four deionized water washes and dried to constant weight to give 5.35 g or a 84.8 % yield of ca. 97 % pure 8-methoxy-4-*H*-1,3-benzodioxin-6-carboxylic acid, m.p. 172-200°C, mostly 191.5-200.0°C.

### Example 5

### Preparation of 5-chloro-4-H-1,3-benzodioxin-6-carboxylic acid and of 7-chloro-4-H-1,3-benzodioxin-6-carboxylic acid using methyl 2-chloro-4-hydroxybenzoate.

This Example illustrates the preparation of 5-chloro-4-*H*-1,3-benzodioxin-6-carboxylic acid and of 7-chloro-4-*H*-1,3-benzodioxin-6-carboxylic acid using methyl 2-chloro-4-hydroxybenzoate, and also illustrates the preparation of the methyl 2-chloro-4-hydroxybenzoate from the corresponding acid.

### (i). Preparation of methyl 2-chloro-4-hydroxybenzoate.

2-Chloro-4-hydroxybenzoic acid (5.22 g, 30.2 mmoles) (Eastman Kodak) was converted to methyl 2-chloro-4-hydroxybenzoate (5.52 g, ca. 29.6 mmoles) by adding thionyl chloride to a methanol solution of the acid and heating.

### (ii). Preparation of 5-chloro-4-H-1,3-benzodioxin-6-carboxylic acid and of 7-chloro-4-H-1,3-benzodioxin-6-carboxylic acid using methyl 2-chloro-4-hydroxybenzoate.

Methyl 2-chloro-4-hydroxybenzoate (5.52 g, 29.6 mmoles), paraformaldehyde (2.90 g, 96.6 mmoles as formaldehyde monomer), 22.77 g toluene, and xylenes-dried p-toluenesulfonic acid (0.66 g, 3.83 mmoles; originally 0.72 g p-toluenesulfonic acid monohydrate) were heated with stirring in a loosely capped 125 ml screw top erlenmeyer flask for 23 minutes at 89-99°C and 13 minutes at 99-103.5°C. Then solid was scraped off the walls of the flask and heating was continued for 87 minutes at 99-103.5°C. The cooled organic layer was decanted from a small amount of insoluble solid and 1.35 g of aqueous p-toluenesulfonic acid with two (3.4 g, 2.0 g) toluene washes. The combined organic supernates were evaporated in a Rotovap evaporator to 7.61 g brown oil. GC/MS of a dilute solution of this oil gave 43.0 area % of the 7-chloro and 32.8 % of the 5-chloro isomer of methyl chloro-4-*H*-1,3-benzodioxin-6-carboxylate. The oil was dissolved in 13.05 g methanol and heated near reflux for five minutes with 50.3 wt. % aqueous sodium hydroxide (3.02 g, 38.0 meq). Deionized water (2.72 g) was added and heating and stirring continued for 3 minutes. Then 2.85 g additional deionized water was added and heating was continued for one hour at 67-73°C. The cooled solution was filtered through glass wool with a 2.35 g methanol/1.99 g deionized water wash and a 2.1 g deionized water wash. Then 13.18 g of solvent was evaporated, 14.76 g deionized water was added, and neutral impurities were extracted with two ca. 10 g diethyl ether washes. The extracted aqueous layer was partially acidifed with 37 % aqueous hydrochloric acid (1.51 g, 15.3 meq) and extracted twice with diethyl ether to remove acidic impurities of higher molecular weight. Then the aqueous layer was acidifed to pH 3 and filtered with four 10 g deionized water washes. Drying to constant weight gave crude mixed 5- and 7-chloro-4-*H*-1,3-benzodioxin-6-carboxylic acids (4.92 g, 20.4 mmoles, 75.9 % yield).

The mixture of crude chloro-4-*H*-1,3-benzodioxin-6-carboxylic acid isomers was separated by column chromatography (50/50 wt./wt. diethyl ether/hexanes on silica gel) and fractional crystallizations from acetone to give 5-chloro-4-*H*-1,3-benzodioxin-6-carboxylic acid (1.11 g, 17.1 % yield),.m.p. 205.2-211.5°C, and 7-chloro-4-*H*-1,3-benzodioxin-6-carboxylic acid (1.32 g, 20.4 %yield), m.p. 217.3-221.2°C, along with recovered mixed isomers (1.30 g, 20.1 % yield).

### Example 6.

### Preparation of 5-chloro-4-H-1,3-benzodioxin-6-carboxylic acid and of 7-chloro-4-H-1,3-benxodioxin-6-carboxylic acid.

This Example illustrates several methods for preparing 5-chloro-4-*H*-1,3-benzodioxin-6-carboxylic acid and of 7-chloro-4-*H*-1,3-benzodioxin-6-carboxylic acid.

### (i). Preparation of 5-chloro-4-H-1,3-benzodioxin-6-carboxylic acid and of 7-chloro-4-H-1,3-benzodioxin-6-carboxylic acid using 2-chloro-4-hydroxybenzoic acid and p-toluenesulfonic acid as catalyst.

2-chloro-4-hydroxybenzoic acid (2.63 g, 15.3 mmoles) (Eastman Kodak), paraformaldehyde (1.55 g, 51.6 mmoles as formaldehyde monomer), and p-toluenesulfonic acid monohydrate catalyst (0.49 g, 2.84 mmoles) were heated with stirring in 13.74 g toluene in a 300 ml single neck round bottom (SNRB) flask for ten minutes at 92.4-99.3°C, 56 minutes at 99.3-109.5°C (sublimed and splashed solid scraped back into liquid) and 66 minutes at 109.5-100.5°C. GC/MS of the hot toluene supernate gave 43.8 area % 5-chloro and 35.3 % 7-chloro-4-*H*-1,3-benzodioxin-6-carboxylic acid along with numerous minor GC peaks. The cooled reaction mixture was partitioned between 25.6 g deionized water containing 50.3 wt. % aqueous sodium hydroxide (1.51 g, 19.0 meq) and 14 g diethyl ether. The aqueous layer was removed and the ether layer was extracted with two small dilute aqueous sodium hydroxide washes and one water wash. The combined aqueous layers were acidified with 37 % aqueous hydrochloric acid (1.80 g or 18.3 meq) and filtered with four 5 g water washes. Drying to constant weight gave 2.89 g or an 88 % yield of ca. 93 % pure mixed chloro-4-*H*-1,3-benzodioxin-6-carboxylic acid isomers (50.0 % 5-chloro and 43.2 % 7-chloro by GC/MS).

### (ii). Preparation of 5-chloro-4-H-1,3-benzodioxin-6-carboxylic acid and of 7-chloro-4-H-1,3-benzodioxin-6-carboxylic acid using 2-chloro-4-hydroxybenzoic acid and methanesulfonic acid as catalyst.

2-chloro-4-hydroxybenzoic acid (2.66 g, 15.4 mmoles) (Eastman Kodak), paraformaldehyde (2.90 g, 96.6 mmoles as monomer), 99.5 g methanesulfonic acid (0.37 g, 3.85 mmoles) and 11.69 g o-dichlorobenzene were heated with stirring in a capped 125 ml erlenmeyer flask for 36 minutes at 110.0-112.7°C. Then the walls of the flask were scraped down and heating was continued for 24 minutes without stirring. The cooled reaction mixture was diluted with 18 g diethyl ether and the organic layer was removed from 4.96 g of moist solid and oil. The organic layer was extracted with 48.3 g deionized water containing 50 % aqueous sodium hydroxide (2.49 g, 31.3 meq), then recycled to a second extraction of the reaction paste. This organic extract was extracted with the same aqueous base solution, as was a final extract of the reaction paste with 26 g diethyl ether. Then they were washed with 10.3 g deionized water containing 0.15 g 50 % sodium hydroxide and discarded. The combined aqueous layers were acidified with 3.20 g 37 % aqueous hydrochloric acid to pH 3-4. The resulting slurry was filtered with five 5 g deionized water washes and the wet cake was dried to constant weight to give mixed chloro-*4*-*H*-1,3-benzodioxin-6-carboxylic acids (2.78 g, 84 % yield) as a light tan solid, m.p. 157-171°C. GC/MS of an acetone solution of the isolated product gave 54.8 % 5-chloro and 43.4 % 7-chloro isomer with 1.8 % of minor components.

### (iii). Preparation of 5-chloro-4-H-1,3-benzodioxin-6-carboxylic acid and of 7-chloro-4-H-1,3-benzodioxin-6-carboxylic acid using boron trifluroide etherate as catalyst

2-Chloro-4-hydroxybenzoic acid (2.63 g, 15.2 mmoles) (Eastman Kodak), paraformaldehyde (1.56 g, 52.0 mmoles as formaldehyde monomer), boron trifluoride etherate (0.23 g, 1.62 mmoles), and 8.78 g o-dichlorobenzene were heated with stirring in a 125 ml erlenmeyer flask for 16 minutes at 67-90°C and 39 minutes at 90-105°C. The sublimed solid was scraped off the walls and heating was continued for 51 minutes at 103-104°C. Then the mixture was cooled and partitioned between 16 g diethyl ether and 23 g deionized water containing 50 % aqueous sodium hydroxide (2.11 g, 26.5 meq). The aqueous layer was removed and the organic layer was washed with three portions of dilute sodium hydroxide (total base 0.41 g or 5.16 meq) until the pH of the wash reached 10-12, then with 4 g deionized water. The aqueous layers were filtered through glass wool to remove traces of solid, combined, and acidified to pH 2 with 37 % aqueous hydrochloric acid (3.48 g, 35.3 meq). The resulting slurry was filtered with four 5 g deionized water washes and dried to constant weight to give crude mixed chloro-4-*H*-1,3-benzodioxin-6-carboxylic acids (2.04 g). GC/MS of an acetone solution of the isolated product gave 22.5 area % 5-chloro and 20.5 % 7-chloro isomer along with 44.9 % unreacted 2-chloro-4-hydroxybenzoic acid and 9.0 % 3-chlorophenol and trace peaks.

### (iv). Preparation of 5-chloro-4-H-1,3-benzodioxin-6-carboxylic acid and of 7-chloro-4-H-1,3-benzodioxin-6-carboxylic acid using 2-chloro-4-hydroxybenzoic acid and 1,3,5-trioxane with methanesulfonic acid catalyst.

2-chloro-4-hydroxybenzoic acid (0.89 g, 5.16 mmoles) (Eastman Kodak), 1,3,5-trioxane (0.58 g, 19.3 mmole as monomer), 99 % methanesulfonic acid (0.04 g, 0.42 mmole) and 3.98 g o-dichlorobenzene were heated with magnetic stirring in a capped 6 dram vial for 72 minutes at 99-116 °C. Then the two liquid phases were partially cooled and partitioned between 7.58 g deionized water containing 50.3 wt % aqueous sodium hydroxide (0.23 g, 2.88 meq) and 37 % aqueous hydrochloric acid (0.03 g, 0.30 meq - emulsion problem decreased with the addition of the hydrochloric acid). After the mixture stood about three days, the aqueous layer was removed and the organic layer was washed with (1) 2.82 g deionized water containing 0.02 g 50% aqueous sodium hydroxide, (2) 2.01 g deionized water containing 0.06 g 50 % sodium hydroxide, and (3) 2.22 g deionized water. The combined extracts were acidified with 37 % aqueous hydrochloric acid (0.34 g, 3.45 meq), giving a tan precipitate. The resulting slurry was extracted with (1) 4.75 g tetrahydrofuran/4.16 g anhydrous diethyl ether, (2) 1.07 g diethyl ether, and (3) 1.03 g diethyl ether. The three extracts were blown down to constant weight in a tared vial and the solid crust was ground to give 0.68 g or a 61.4 % yield of crude (ca. 90 % pure, impurity is primarily tetrahydrofuran solvent) mixed chloro-*4*-*H*-1,3-benzodioxin-6-carboxylic acid isomers (46.9 % 5-chloro and 52.1 % 7-chloro by GC/MS).

Another 0.30 g or 27.1 % yield of crude mixed chloro-4-*H*-1,3-benzodioxin-6-carboxylic acid isomers of lower purity (75 %, impurities primarily tetrahydrofuran (20 % by weight) and o-dichloro-benzene) was recovered from the original organic layer by further extracting it with (4) 2.13 g deionized water containing 0.15 g 50 % aqueous sodium hydroxide, (5) 1.28 g deionized water, (6) 2.42 g deionized water containing 0.02 g 50 % aqueous sodium hydroxide, and (7) 1.67 g deionized water containing 0.05 g 50 % aqueous sodium hydroxide (aqueous layer first strongly basic). The extracts were combined, acidified and extracted as above. Then the extracts were evaporated to give the second crop of mixed chloro-4-*H*-1,3-benzodioxin-6-carboxylic acid isomers (49.1 area % 5-chloro and 43.3 % 7-chloro by GC/MS).

The following Examples illustrate the preparation of a number of N-benzoyl-N-tert-butyl-N'-1,3-benzodioxincarbonyl hydrazines and their use as insecticides.

### Example 7

### Preparation of N-3,5-dimethylbenzoyl-N'-8-methyl-4-H-1,3-benzodioxin-7-carbonyl-N-tert-butylhydrazine.

Purified 8-methyl-4-*H*-1,3-benzodioxin-7-carboxylic acid (0.78 g, 4.02 mmoles), was converted to the acid chloride (0.88 g) by heating in a loosely capped 4 dram vial with excess thionyl chloride and N,N-dimethylformamide catalyst. Residual thionyl chloride was removed by adding and evaporating toluene. Then the acid chloride was dissolved in 4.85 g propyl acetate and N-3,5-dimethylbenzoyl-N-tert-butylhydrazine (0.95 g, 4.31 mmoles) was added with shaking. The slurry was stirred for 50 minutes at room temperature. Then 41.3 wt. % aqueous potassium carbonate solution (2.37 g, 7.08 mg form. wt.) was added with shaking. After the vial stood for 69 hours at room temperature with six shakings during the first 3.6 hours, the mixture was filtered with three 1.6-2.7 g diethyl ether washes and four 3.6-5.2 g deionized water washes. Drying gave crude N-3,5-dimethylbenzoyl-N'-8-methyl-4-*H*-1,3,-benzodioxin-7-carbonyl-N-tert-butylhydrazine (1.4 g, 88 % yield) as tan crystals, m.p. 192-199°C. This solid was dissolved in 5.29 g hot methanol, 1.73 g deionized water was added with reheating, and the mixture was allowed to crystallize with eventual cooling to -10 to -15°C. Filtration with a 0.98 g methanol/0.63 g deionized water wash and a 2.19 g methanol/1.28 g deionized water wash and drying gave purified N-3,5-dimethylbenzoyl-N'-8-methyl-4-*H*-1,3,-benzodioxin-7-carbonyl-N-tert-butylhydrazine (0.93 g) as an off-white solid, m.p. 200.2-205.7°C.

### Examples 8 to 22.

The following additional N-benzoyl -N-tert-butyl-N'-1,3-benzodioxin-carbonyl hydrazines were prepared by methods similar to that described in Example 7.

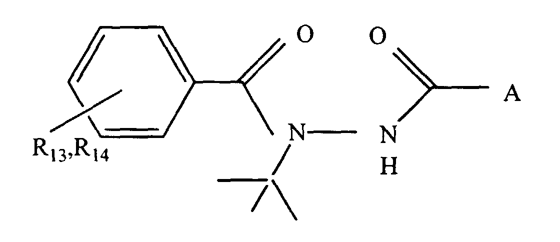

| Example | R₁₃,R₁₄ | A | m.p., °C |
|---|---|---|---|
| 8 | 3,5-Me₂ | 4-*H*-1,3-benzodioxin-7-carbonyl | 200.2-203.2 |
| 9 | 2-Cl | 4-*H*-1,3-benzodioxin-7-carbonyl | 205.0-210.1 |
| 10 | H | 4-*H*-1,3-benzodioxin-7-carbonyl | 231.6-235.2 |
| 11 | 3-Me | 8-Me-4-*H*-1,3-benzodioxin-7-carbonyl | 200.2-205.7 |
| 12 | 2-OMe | 8-Me-4-*H*-1,3-benzodioxin-7-carbonyl | 124.2-127.2 |
| 13 | 4-F | 8-Me-4-*H*-1,3-benzodioxin-7-carbonyl | 213-223 |
| 14 | 3-OMe | 8-Me-4-*H*-1,3-benzodioxin-7-carbonyl | 213.8-215-0 |
| 15 | H | 8-Me-4-*H*-1,3-benzodioxin-7-carbonyl | 122.4-128.0 |
| 16 | 3,5-Me₂ | 8-Et-4-*H*-1,3-benzodioxin-7-carbonyl | 165.0-169.0 |
| 17 | 3-Me | 8-Et-4-*H*-1,3-benzodioxin-7-carbonyl | 222.2-225.0 |
| 18 | 4-F | 8-Et-4-*H*-1,3-benzodioxin-7-carbonyl | 242.5-249.2 |
| 19 | 3,5-Me₂ | 5-Cl-4-*H*-1,3-benzodioxin-6-carbonyl | 224.5-227.1 |
| 20 | H | 5-Cl-4-*H*-1,3-benzodioxin-6-carbonyl | 211.2-212.9 |
| 21 | H | 7-Cl-4-*H*-1,3-benzodioxin-6-carbonyl | 205.5-207.5 |
| 22 | 3,5-Me₂ | 7-Cl-4-*H*-1,3-benzodioxin-6-carbonyl | 208.4-212.3 |
| 23 | 3,5-Me₂ | 4-*H*-1,3-benzodioxin-6-carbonyl | 215-222.2 |

The following Example 24 illustrates the insecticidal activity of a number of N-benzoyl-N-tert-butyl-N'-1,3-benzodioxincarbonyl hydrazines.

### Example 24.

Whole insect activity against Southern Armyworm (AW) (*spodoptera eridania*) and especially tobacco budworm (TBW) (*Heliothis virescens*) was observed to generally increase with 8-alkyl substitution (see Table I below, for example, Examples 7 and 16 versus Example 8, and Example 15 versus Example 10).

The evaluation of the compounds of the invention was carried out as follows:-

A test solution containing 600 ppm was made by dissolving a compound of the invention in a 1:1 acetone:methanol solution, then adding water to give a 5:5:90 acetone:methanol:water solution, and finally a surfactant was added at an equivalent of 7.37 g of surfactant per 100 liters of test solution (1 ounce of surfactant per 100 gallons of test solution). Appropriate dilutions were prepared in water from the 600 ppm solution.

### For the Southern Armyworm:

An individual lima bean leaf, *Phaseolus limensis* var. Woods' Prolific, was placed on moistened filter paper in a Petri dish (100 x 20 mms). The leaf was sprayed with the test solution using a rotating turntable sprayer and allowed to dry. The dish was infested with 10 third instar larvae of the Southern Armyworm and covered with the lid. If the larvae were alive two days after treatment, fresh untreated bean leaves were added. All treatments were maintained at 23.9-26.7°C (75-80°F) under fluorescent light in a well ventilated room. Percent mortality was determined at four days after treatment.

### For Tobacco Budworm:

The same procedure was used as for the Southern Armyworm except that a detached cotton leaf, *Gossypium hirsutum*, was used with first instar larvae.

**Table 1**

| LC50s, ppm | | |
|---|---|---|
| Example | AW | TBW |
| | | |

| A equals 4-*H*-1,3-benzodioxin-7-carbonyl | | |
|---|---|---|
| 8 | 3.0 | 62 |
| 9 | 150 | NT |
| 10 | 62 | NT |

| A equals 8-methyl-4-*H*-1,3-benzodioxin-7-carbonyl | | |
|---|---|---|
| 7 | 2.5 | 31 |
| 11 | 10 | 150 |
| 12 | 10 | 10 |
| 13 | 5.0 | 10 |
| 14 | 10 | >600 |
| 15 | <2.5 | 10 |

| A equals 8-ethyl-4-*H*-1,3-benzodioxin-7-carbonyl | | |
|---|---|---|
| 16 | <0.15 | 10 |
| 17 | 10 | 150 |
| 18 | 78 | 360 |

| A equals 5-Cl-4-*H*-1,3-benzodioxin-6-carbonyl | | |
|---|---|---|
| 19 | 10 | 10 |
| 20 | 10 | 4.2 |

| A equals 7-Cl-4-*H*-1,3-benzodioxin-6-carbonyl | | |
|---|---|---|
| 21 | >600 | >600 |
| 22 | 25 | 740 |

| A equals 4-*H*-1,3-benzodioxin-6-carbonyl | | |
|---|---|---|
| 23 | 5.0 | >600 |
| "NT" means not tested on this species. | | |

## Claims

1. A compound having the structural formula: 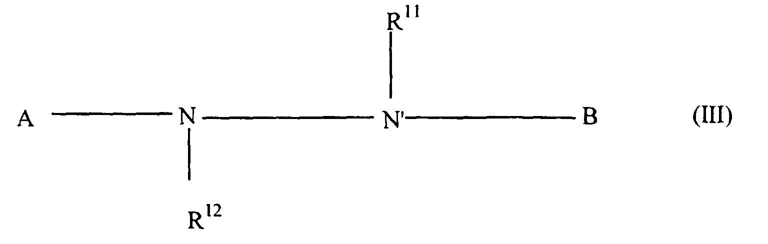 wherein:
(i). one of R¹¹ and R¹² is hydrogen and the other of R¹¹ and R¹² is unsubstituted (C₃-C₁₀)branched alkyl or a (C₁-C₄)straight chain alkyl substituted with one or two of the same or different (C₃-C₆)cycloalkyl;
(ii). A is: 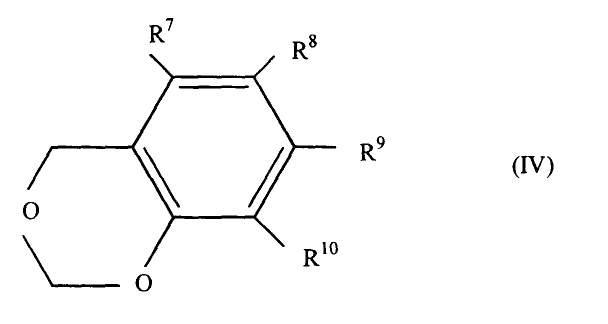 wherein:
one of R⁷, R⁸ or R⁹ is a -C(O)- group, and R¹⁰ and the others of R⁷, R⁸ and R⁹ are independently selected from hydrogen, (C₁-C₂₀)alkyl, halo, halo(C₁-C₂₀)alkyl and (C₁-C₂₀)alkoxy;
provided that at least one of R⁷, R⁸, R⁹ and R¹⁰ is hydrogen; and
(iii) B is -C(O)-Z -
wherein Z is:
phenyl;
naphthyl; or
phenyl or naphthyl substituted with one to three of the same or different substituents selected from the group consisting of: halo; cyano; nitro; hydroxy; mercapto; thiocyanato; (C₁-C₄)alkyl; (C₁-C₄)alkoxy; halo(C₁-C₂)alkyl; halo(C₁-C₂)alkoxy; (C₁-C₄)alkylthio; (C₁-C₄)alkylsulfinyl; (C₁-C₄)alkylsulfonyl; carboxy; formyl; (C₁-C₄)alkylcarbonyl; (C₁-C₄)alkoxycarbonyl; (C₁-C₄)alkanoyloxy; amino; (C₁-C₄)alkylamino; di(C₁-C₄)alkylamino having independently the stated number of carbon atoms in each alkyl group; carbamoyl; (C₁-C₄)alkylcarbamoyl; di(C₁-C₄)alkylcarbamoyl having independently the stated number of carbon atoms in each alkyl group; cyano(C₁-C₄)alkyl; (C₁-C₄)alkoxy(C₁-C₄)alkyl; (C₂-C₆)alkenyl; (C₄-C₆)alkadienyl; (C₂-C₆)alkynyl; (C₁-C₄)alkyldithionate; (C₁-C₄)alkylcarbonylthio; tri(C₁-C₄)alkylsilyl having independently the stated number of carbon atoms in each alkyl group; phenyl; phenyl substituted with one to two of the same or different substituents selected from the group consisting of halo, cyano, nitro, hydroxy, (C₁-C₄)alkyl, (C₁-C₄)alkoxy, halo(C₁-C₂)alkyl, halo(C₁-C₂)alkoxy, (C₁-C₄)alkylthio, (C₁-C₄)alkylsulfinyl, (C₁-C₄)alkylsulfonyl, carboxy, formyl, (C₁-C₄)alkylcarbonyl, (C₁-C₄)alkoxycarbonyl, (C₁-C₄)alkanoyloxy, amino, (C₁-C₄)alkylamino, di(C₁-C₄)alkylamino having independently the stated number of carbon atoms in each alkyl group; phenoxy; phenoxy substituted with one to two of the same or different substituents selected from the group consisting of halo, cyano, nitro, hydroxy, (C₁-C₄)alkyl, (C₁-C₄)alkoxy, halo(C₁-C₂)alkyl, halo(C₁-C₂)alkoxy, (C₁-C₄)alkylthio, (C₁-C₄)alkylsulfinyl, (C₁-C₄)alkylsulfonyl, carboxy, formyl, (C₁-C₄)alkylcarbonyl, (C₁-C₄)alkoxycarbonyl, (C₁-C₄)alkanoyloxy, amino, (C₁-C₄)alkylamino, di(C₁-C₄)alkylamino having independently the stated number of carbon atoms in each alkyl group; benzoyl; benzoyl substituted with one to two of the same or different substituents selected from the group consisting of halo, cyano, nitro, hydroxy, (C₁-C₄)alkyl, (C₁-C₄)alkoxy, halo(C₁-C₂)alkyl, halo(C₁-C₂)alkoxy, (C₁-C₄)alkylthio, (C₁-C₄)alkylsulfinyl, (C₁-C₄)alkylsulfonyl, carboxy, formyl, (C₁-C₄)alkylcarbonyl, (C₁-C₄)alkoxycarbonyl, (C₁-C₄)alkanoyloxy, amino, (C₁-C₄)alkylamino, di(C₁-C₄)alkylamino having independently the stated number of carbon atoms in each alkyl group; phenoxycarbonyl; phenoxycarbonyl substituted with one to two of the same or different substituents selected from the group consisting of halo, cyano, nitro, hydroxy, (C₁-C₄)alkyl, (C₁-C₄)alkoxy, halo(C₁-C₂)alkyl, halo(C₁-C₂)alkoxy, (C₁-C₄)alkylthio, (C₁-C₄)alkylsulfinyl, (C₁-C₄)alkylsulfonyl, carboxy, formyl, (C₁-C₄)alkylcarbonyl, (C₁-C₄)alkoxycarbonyl, (C₁-C₄)alkanoyloxy, amino, (C₁-C₄)alkylamino, di(C₁-C₄)alkylamino having independently the stated number of carbon atoms in each alkyl group; phenylthio; phenylthio substituted with one to two of the same or different substituents selected from the group consisting of halo, cyano, nitro, hydroxy, (C₁-C₄)alkyl, (C₁-C₄)alkoxy, halo(C₁-C₂)alkyl, halo(C₁-C₂)alkoxy, (C₁-C₄)alkylthio, (C₁-C₄)alkylsulfinyl, (C₁-C₄)alkylsulfonyl, carboxy, formyl, (C₁-C₄)alkylcarbonyl, (C₁-C₄)alkoxycarbonyl, (C₁-C₄)alkanoyloxy, amino, (C₁-C₄)alkylamino, di(C₁-C₄)alkylamino having independently the stated number of carbon atoms in each alkyl group; phenyl(C₁-C₄)alkyl; phenyl(C₁-C₄)alkyl substituted on the phenyl ring with one to two of the same or different substituents selected from the group consisting of halo, cyano, nitro, hydroxy, (C₁-C₄)alkyl, (C₁-C₄)alkoxy, halo(C₁-C₂)alkyl, halo(C₁-C₂)alkoxy, (C₁-C₄)alkylthio, (C₁-C₄)alkylsulfinyl, (C₁-C₄)alkylsulfonyl, carboxy, formyl, (C₁-C₄)alkylcarbonyl, (C₁-C₄)alkoxycarbonyl, (C₁-C₄)alkanoyloxy, amino, (C₁-C₄)alkylamino, di(C₁-C₄)alkylamino having independently the stated number of carbon atoms in each alkyl group; or when two adjacent positions on a phenyl ring are substituted with alkoxy groups, these groups may be joined to form a 5 or 6 membered dioxolano (methylenedioxy) or dioxano (1,2-ethylenedioxy) heterocyclic ring.

2. The compound of claim 1, wherein R¹² is hydrogen.

3. The compound of claim 2, wherein R¹¹ is t-butyl.

4. The compound of claim 1, wherein:
(i) one of R⁷, R⁸ and R⁹ is -C(O)-, and R¹⁰ and the others of R⁷, R⁸ and R⁹ are independently selected from hydrogen, (C₁-C₄)alkyl, halo, halo(C₁-C₄)alkyl and (C₁-C₄)alkoxy.

5. The compound of claim 4, wherein:
(i) R⁷ and R⁸ are hydrogen;
(ii) R⁹ is -C(O)-; and
(iii) R¹⁰ is selected from hydrogen (C₁-C₄)alkyl and halo(C₁-C₄)alkyl.

6. The compound of claim 5, wherein R¹⁰ is selected from methyl and ethyl.

7. The compound of claim 4, wherein:
(i) R⁷ and R⁹ are independently selected from (C₁-C₄)alkyl, halo, halo(C₁-C₄)alkyl, and (C₁-C₄)alkoxy;
(ii) R⁸ is -C(O)-; and
(iii) R¹⁰ is hydrogen.

8. A compound having the structural formula (I): 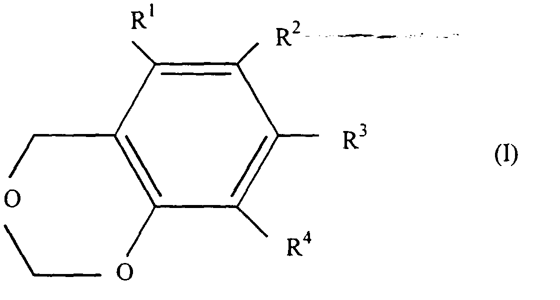 wherein:
one of R¹, R² and R³ is -COOR⁵ wherein R⁵ is selected from hydrogen, (C₁-C₂₀)alkyl, and halo(C₁-C₂₀)alkyl, and R⁴ and the others of R¹, R² and R³ are independently selected from hydrogen, (C₁-C₂₀)alkyl, halo, halo(C₁-C₂₀)alkyl and (C₁-C₂₀)alkoxy;
provided that: (a) at least one of R¹, R², R³ and R⁴ is hydrogen; (b) the compound of formula (I) is not an unsubstituted 4-*H*-1,3-benzodioxin-6-carboxylic acid or ester, and (c) when R¹ and R³ are hydrogen, and R² is -COOR⁵, R⁴ is not halo.

9. The compound of claim 8, wherein:
(ii) one of R¹, R² and R³ is -COOR⁵ wherein R⁵ is selected from hydrogen, (C₁-C₄)alkyl, and halo(C₁-C₄)alkyl, and R⁴ and the others of R¹, R² and R³ are independently selected from hydrogen, (C₁-C₄)alkyl, halo, halo(C₁-C₄)alkyl and (C₁-C₄)alkoxy.

10. The compound of claim 9, wherein:
(i) R¹ and R² are hydrogen;
(ii) R³ is -COOH; and
(iii) R⁴ is selected from hydrogen, (C₁-C₄)alkyl, and halo(C₁-C₄)alkyl.

11. The compound of claim 10, wherein R⁴ is selected from methyl and ethyl.

12. The compound of claim 9, wherein:
one of R¹ and R³ is selected from (C₁-C₄)alkyl, halo, halo(C₁-C₄)alkyl, and (C₁-C₄)alkoxy;
(ii) R² is -COOH; and
(iii) R⁴ and the other of R¹ and R³ are hydrogen.

13. A process for preparing a compound having the structural formula (I): 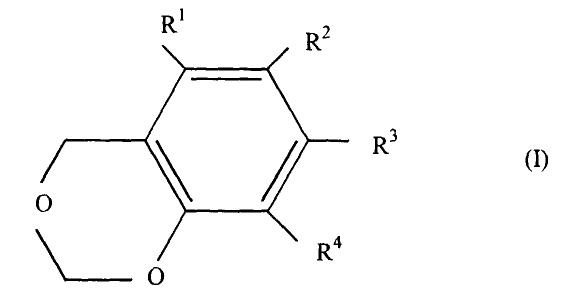 wherein:
(i) one of R¹, R² and R³ is -COOR⁵ wherein R⁵ is selected from hydrogen,(C₁-C₂₀)alkyl, and halo(C₁-C₂₀)alkyl, and R⁴ and the others of R¹, R² and R³ are independently selected from hydrogen, (C₁-C₂₀)alkyl, halo, halo(C₁-C₂₀)alkyl and (C₁-C₂₀)alkoxy;
provided that: (a) at least one of R¹, R², R³ and R⁴ is hydrogen; (b) the compound of formula (I) is not an unsubstituted 4-*H*-1,3-benzodioxin-6-carboxylic acid or ester, and (c) when R² is -COOR⁵, R⁴ is not halo, the process comprising:
reacting a hydroxybenzoic acid or ester having the structural formula (II): 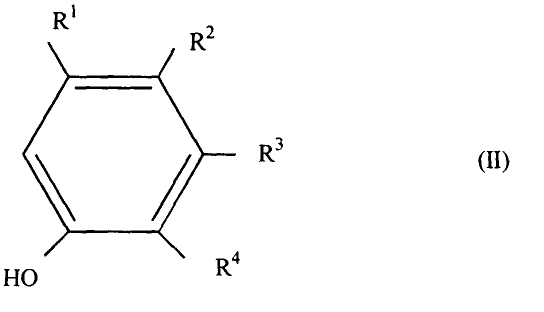 wherein R¹, R², R³, and R⁴ are as defined above
with substantially dry formaldehyde gas or a substantially dry formaldehyde-generating compound in the presence of a Lewis acid or Bronsted acid catalyst, and wherein the level of water during the reaction does not exceed 30 weight percent based on the weight of the hydroxybenzoic acid or ester.

14. The process of claim 13, wherein the reaction is carried out in the presence of a non-reactive organic solvent.

15. The process of claim 13, wherein R⁵ is selected from (C₁-C₂₀)alkyl, and halo(C₁-C₂₀)alkyl, further comprising hydrolysing R⁵ with a strong base in the presence of water or a wet organic solvent to produce the compound of formula (I) wherein R⁵ is hydrogen.

16. The process of claim 13, wherein the dry formaldehyde-generating compound is selected from paraformaldehyde and 1,3,5-trioxane.

17. An insecticidal composition, which comprises an N'-substituted-N,N'-diacylhydrazine according to claim 1 and an agronomically acceptable carrier.

18. A method of controlling insects, which comprises applying to a plant, to the locus of a plant, or to seeds, an N'-substituted-N,N'-diacylhydrazine as claimed in claim 1.

19. A method of controlling insects, which comprises applying to a plant, or to the locus of a plant, or to seeds, an insecticidal composition as claimed in claim 17.
